# EUROPÄISCHE PATENTANMELDUNG

(11) **EP 4 702 969 A1**
(43) Veröffentlichungstag der Anmeldung: **04.03.2026**
(21) Anmeldenummer: 24197772.7
(22) Anmeldetag: 30.08.2024
(51) Int. Cl.: A61K 8/49, A61Q 5/06, A61K 8/34

(54) **MITTEL ZUM FÄRBEN VON KERATINFASERN**

(71) Anmelder: Dr. Kurt Wolff GmbH & Co. KG, 33611 Bielefeld (DE)
(72) Erfinder: SCHULZE ZUR WIESCHE, Erik, 33611 Bielefeld (DE); HAGENSTEIN, Miriam, 33611 Bielefeld (DE)
(74) Vertreter: Weickmann & Weickmann PartmbB

(57) **Zusammenfassung**

Die vorliegende Erfindung betrifft eine kosmetische Formulierung enthaltend ein Farbstoffvorprodukt, einen direktziehenden Farbstoff und mindestens 8 Gew.-% eines aliphatischen Kohlenwasserstoffs mit mindestens 10 Kohlenstoffatomen, dessen Verwendung zum Färben von Keratinfasern und ein entsprechendes Anwendungsverfahren. Die Erfindung betrifft ferner einen Kit umfassend die erfindungsgemäße kosmetische Formulierung in Kombination mit einer zusätzlichen kosmetischen Formulierung, wie zum Beispiel ein Shampoo.

## Beschreibung

Die vorliegende Erfindung betrifft eine kosmetische Formulierung enthaltend ein Farbstoffvorprodukt, einen direktziehenden Farbstoff und mindestens 8 Gew.-% eines aliphatischen Kohlenwasserstoffs mit mindestens 10 Kohlenstoffatomen, dessen Verwendung zum Färben von Keratinfasern und ein entsprechendes Anwendungsverfahren. Die Erfindung betrifft ferner einen Kit umfassend die erfindungsgemäße kosmetische Formulierung in Kombination mit einer zusätzlichen kosmetischen Formulierung, wie zum Beispiel ein Shampoo.

Die Haarfarbe eines Menschen hängt von der Menge der Melanine Eumelanin und Phäomelanin ab, die in der Faserschicht der Haare enthalten sind und von Melanozyten in den Haarfollikeln produziert werden. Im Rahmen des normalen Alterungsprozesses, aber auch bedingt durch bestimmte Krankheitszustände, Stress, Vitamin- und Mineralstoffmangel oder übermäßigen Alkohol- oder Nikotinkonsum kann es zum Nachlassen der Melaninproduktion kommen, in deren Folge es zu einer Hypopigmentierung kommt. Solche Haare erscheinen dem Betrachter weiß oder farblos. Der optische Eindruck von grauem Haar resultiert aus der Mischung aus pigmentierten und pigmentlosen Haaren.

Der durchschnittliche Lebenszyklus eines Haars beträgt drei bis sieben Jahre, dann fallen die Haare aus und ein neues Haar wächst an dieser Stelle. Allmählich überwiegt der Anteil an weißen oder pigmentlosen Haaren, das Haar ergraut.

Die Veränderung der Haarfarbe und insbesondere die Pigmentierung oder Repigmentierung von grauen Haaren stellt einen wichtigen Bereich der modernen Kosmetik dar. Hierbei sind insbesondere gute Echtheitseigenschaften und gute Grauabdeckung sowie langanhaltende Dauer der Färbungen wünschenswert.

Für permanente, intensive Färbungen mit guten Echtheitseigenschaften und guter Grauabdeckung werden herkömmlicherweise Oxidationsfärbemittel verwendet.

Solche Färbemittel enthalten üblicherweise Oxidationsfarbstoffvorprodukte, sogenannte Entwicklerkomponenten und Kupplerkomponenten, die unter dem Einfluss von Oxidationsmitteln, wie z.B. Wasserstoffperoxid, oder von Luftsauerstoff untereinander oder unter Kupplung mit einer oder mehreren Kupplerkomponenten die eigentlichen Farbstoffe ausbilden.

Der Einsatz oxidativer Färbemittel ist jedoch nach wie vor mit Beeinträchtigungen oder Schädigungen der Haare und insbesondere deren Oberflächenstruktur verbunden.

Alternativ können Vorstufen des natürlichen Haarfarbstoffes Melanin auf das Haar aufgebracht werden, die im Rahmen oxidativer Prozesse im Haar praktisch naturidentische Farbstoffe ausbilden. Die Ausfärbung kann dabei mit Luftsauerstoff als einzigem Oxidationsmittel erfolgen, so dass keine weiteren Oxidationsmittel erforderlich sind. Ein solches Verfahren mit 5,6-Dihydroxyindolin als Farbstoffvorprodukt ist beispielsweise in EP 0 530 229 B1 beschrieben.

Im Gegensatz dazu kommen für temporäre Färbungen direktziehende Farbstoffe, auch Direktzieher genannt, zum Einsatz. Hierbei handelt es sich um Farbstoffmoleküle, die direkt auf das Haar aufziehen und keinen oxidativen Prozess zur Ausbildung der Farbe mehr benötigen. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

WO 99/66890 beschreibt ein Färbemittel zum Färben von Keratinfasern enthaltend ein Farbstoffvorprodukt, bei welchem es sich um ein Indolin- oder Indol-Derivat, z.B. ein Derivat des 5,6-Dihydroxyindolins oder des 5,6-Dihydroxyindols, handeln kann, sowie eine Aminosäure oder ein Oligopeptid. Die Ausbildung der Farbe kann mittels Luftoxidation erfolgen.

DE 10 2018 127 182 A1 betrifft ein Zweikomponentensystem zur künstlichen Haarfärbung enthaltend getrennt voneinander ein wasserfreies Trägermedium, welches ein Alkan, einen Fettalkohol und einen Alkohol umfasst, sowie eine wässrige Phase, welche Wasserstoffperoxid enthält. Eine siliciumorganische Verbindung aus der Gruppe der Silane kann zum Schutz und zur Pflege der Haare enthalten sein.

DE 10 2007 038 484 A1 beschreibt ein Haarbehandlungsmittel zum Schutz vor äußeren Einflüssen, wobei das Haarbehandlungsmittel Bakterienferment(e) aus *Thermus thermophilus* enthält.

WO 2005/007615 A1 betrifft 2-(Amino- oder substituierte Amino)-5-(substituierte oxymethyl)-phenolverbindungen sowie Zusammensetzungen zum oxidativen Färben von Keratinfasern.

EP 4 154 864 beschreibt eine kosmetische Formulierung, welche neben Farbstoffvorprodukten auch einen direktziehenden Farbstoff enthält.

Zusammenfassend besteht insbesondere bei den Echtheitseigenschaften, der Grauabdeckung und der Dauer der Färbeleistung weiterhin Verbesserungsbedarf.

Überraschenderweise wurde gefunden, dass die Ergebnisse hinsichtlich Qualität, Intensität und Dauer der Färbeleistung bei gleichzeitig möglichst geringer Beeinträchtigung oder Schädigung der Haarstruktur verbessert werden, wenn die Formulierung zusätzlich einen gewissen Anteil an ggf. funktionalisierten aliphatischen Kohlenwasserstoffen enthält. Ohne an eine Theorie gebunden zu sein, scheint die Kohlenwasserstoffkomponente ein Einziehen des Farbstoffvorprodukts und/oder des direktziehenden Farbstoffs in die Keratinfaser zu verbessern.

Die Aufgabe der vorliegenden Erfindung kann somit darin gesehen werden, eine kosmetische Formulierung bereitzustellen, die zur Pigmentierung oder Repigmentierung von Keratinfasern, insbesondere menschlichen Haaren, geeignet ist und insbesondere verbesserte Echtheitseigenschaften der pigmentierten oder repigmentierten Keratinfasern sowie eine verbesserte Grauabdeckung bietet. Weiterhin sollte diese kosmetische Formulierung hinsichtlich Qualität, Intensität und Dauer der Färbeleistung verbesserte Eigenschaften gegenüber herkömmlichen Formulierungen aufweisen.

Die Aufgabe wurde überraschend durch eine kosmetische Formulierung gelöst, die
(i) mindestens ein Farbstoffvorprodukt ausgewählt aus der Gruppe bestehend aus Indolin-Derivaten und Indol-Derivaten,
(ii) mindestens einen direktziehenden Farbstoff und
(iii) mindestens einen aliphatischen Kohlenwasserstoff mit mindestens 10, bevorzugt 10-50, Kohlenstoffatomen, der ggf. mit mindestens einer funktionellen Gruppe ausgewählt aus -OH und -COOR substituiert ist, wobei R unabhängig voneinander H, ein Glycerinderivat oder ein physiologisch verträgliches Kation ist,

wobei die Komponente (iii) bevorzugt mindestens 8 Gew.-%, stärker bevorzugt 8-20 Gew.-%, bezogen auf die Gesamtmasse der kosmetischen Formulierung ausmacht,
enthält.

Es wurde überraschenderweise festgestellt, dass graue Haare deutlich abgedeckt und die Haare schrittweise dunkler werden, wobei sich der Farbeffekt dezent aufbaut. Der Gesamteindruck der Haarfarbe wirkt deutlich dunkler. Hinzu kommt, dass auch nach mehrmaligem Haarewaschen die Färbung kaum nachlässt und ein Nachgrauen am Haaransatz reduziert ist.

Das in der erfindungsgemäßen Formulierung enthaltene Farbstoffvorprodukt, bei dem es sich um ein Indol-Derivat und/oder Indolin-Derivat handelt, ist bevorzugt ein Indol-Derivat und/oder Indolin-Derivat, welches eine Hydroxy- oder Aminogruppe, bevorzugt als Substituent am Sechsring aufweist. Diese Gruppen können weitere Substituenten tragen, z. B. in Form einer Veretherung oder Veresterung der Hydroxygruppe oder eine Alkylierung der Aminogruppe. Verbindungen mit zwei dieser Gruppen, insbesondere zwei Hydroxygruppen, von denen eine oder beide verethert oder verestert sein können, sind besonders bevorzugt.

In einer bevorzugten Ausführungsform der Erfindung handelt es sich bei dem Farbstoffvorprodukt um ein Indol-Derivat.

Erfindungsgemäß bevorzugt ist das Farbstoffvorprodukt ein Derivat des 5,6-Dihydroxyindols der Formel (I), 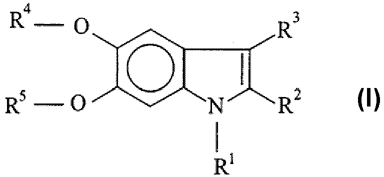 wobei, jeweils unabhängig voneinander,
R¹ Wasserstoff, eine C₁-C₄-Alkylgruppe oder eine C₁-C₄-Hydroxyalkylgruppe bedeutet,
R² Wasserstoff oder eine -COOH-Gruppe bedeutet, wobei die -COOH-Gruppe auch als Salz mit einem physiologisch verträglichen Kation vorliegen kann,
R³ Wasserstoff oder eine C₁-C₄-Alkylgruppe bedeutet,
R⁴ Wasserstoff, eine C₁-C₄-Alkylgruppe, eine Aminogruppe oder eine Gruppe - COR⁶ bedeutet, in der R⁶ eine C₁-C₄-Alkylgruppe bedeutet, und
R⁵ eine der unter R⁴ genannten Gruppen bedeutet,
oder ein physiologisch verträgliches Salz dieser Verbindungen mit einer organischen oder anorganischen Säure.

Bevorzugte Indolderivate sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol.

In einer besonders bevorzugten Ausführungsform ist die Verbindung der Formel (I) ausgewählt aus 5,6-Dihydroxyindol, N-Methyl-5,6-dihydroxyindol sowie deren physiologisch verträgliche Salze.

Ganz besonders bevorzugt handelt es sich bei dem in der erfindungsgemäßen Formulierung eingesetzten Farbstoffvorprodukt um 5,6-Dihydroxyindol.

In einer weiteren bevorzugten Ausführungsform der Erfindung handelt es sich bei dem Farbstoffvorprodukt um ein Indolin-Derivat.

Bevorzugt ist das Farbstoffvorprodukt ein Derivat des 5,6-Dihydroxyindolins der Formel (II), 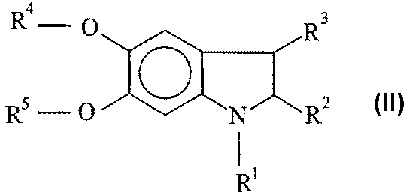 wobei, jeweils unabhängig voneinander,
R¹ Wasserstoff, eine C₁-C₄-Alkylgruppe oder eine C₁-C₄-Hydroxyalkylgruppe bedeutet,
R² Wasserstoff oder eine -COOH-Gruppe bedeutet, wobei die -COOH-Gruppe auch als Salz mit einem physiologisch verträglichen Kation vorliegen kann,
R³ Wasserstoff oder eine C₁-C₄-Alkylgruppe bedeutet,
R⁴ Wasserstoff, eine C₁-C₄-Alkylgruppe, eine Aminogruppe oder eine Gruppe - COR⁶ bedeutet, in der R⁶ eine C₁-C₄-Alkylgruppe bedeutet, und
R⁵ eine der unter R⁴ genannten Gruppen bedeutet,
oder ein physiologisch verträgliches Salz dieser Verbindungen mit einer organischen oder anorganischen Säure.

Bevorzugte Indolinderivate sind 5,6-Dihydroxyindolin, N-Methyl-5,6-dihydroxyindolin, N-Ethyl-5,6-dihydroxyindolin.

In einer besonders bevorzugten Ausführungsform ist die Verbindung der Formel (II) ausgewählt aus 5,6-Dihydroxyindolin, N-Methyl-5,6-dihydroxyindolin sowie deren physiologisch verträgliche Salze.

Ganz besonders bevorzugt handelt es sich bei dem in der erfindungsgemäßen Formulierung eingesetzten Farbstoffvorprodukt um 5,6-Dihydroxyindolin.

In einer weiteren besonders bevorzugten Ausführungsform handelt es sich bei dem in der erfindungsgemäßen Formulierung eingesetzten Farbstoffvorprodukt um Dihydroxyindolin-hydrobromid (2,3-Dihydro-1H-indol-5,6-diol-hydrobromid).

Die in den erfindungsgemäßen Mitteln enthaltenen Indol- und Indolin-Derivate können sowohl als freie Basen als auch in Form ihrer physiologisch verträglichen Salze mit anorganischen oder organischen Säuren, z. B. der Hydrochloride, der Sulfate und Hydrobromide, eingesetzt werden.

Das Indol- oder Indolin-Derivat ist in der erfindungsgemäßen Formulierung üblicherweise in einer Menge von mindestens 0,1 Gew.-%, bevorzugt mindestens 0,2 Gew.-%, vorzugsweise 0,2-1,5 Gew.-%, bevorzugt 0,2-0,5 Gew.-%, bevorzugt 0,3-0,5 Gew.-%, noch stärker bevorzugt 0,35-0,5 Gew.-%, bevorzugt in einer Menge von etwa 0,2 Gew.-%, 0,25 Gew.-%, 0,3 Gew.-%, 0,35 Gew.-%, 0,4 Gew.-%, 0,5 Gew.-%, jeweils bezogen auf das Gesamtgewicht der Formulierung, enthalten.

In einer besonders bevorzugten Ausführungsform handelt es sich bei dem Farbstoffvorprodukt um ein Indol-Derivat, vorzugsweise 5,6-Dihydroxyindol, wobei dieses in der erfindungsgemäßen Formulierung bevorzugt in einer Menge von 0,2-2 Gew.-%, bevorzugt 0,2-0,5 Gew.-%, jeweils bezogen auf das Gesamtgewicht der Formulierung, enthalten ist.

In einer weiteren besonders bevorzugten Ausführungsform handelt es sich bei dem Farbstoffvorprodukt um ein Indolin-Derivat, vorzugsweise Dihydroxyindolin-hydrobromid (2,3-Dihydro-1H-indol-5,6-diol-hydrobromid), wobei dieses in der erfindungsgemäßen Formulierung bevorzugt in einer Menge von 0,2-1,5 Gew.-%, bevorzugt 0,2-0,5 Gew.-%, bevorzugt 0,3-0,5 Gew.-%, jeweils bezogen auf das Gesamtgewicht der Formulierung, enthalten ist.

Die erfindungsgemäße Formulierung kann auch mehr als ein Indol- oder Indolin-Derivat oder Mischungen von Indol- und Indolin-Derivaten enthalten.

Die Ausbildung des Pigments aus dem Farbstoffvorprodukt, d. h. dem Indol- oder Indolin-Derivat, erfolgt durch Reaktion mit Luftsauerstoff, es ist kein zusätzliches Oxidationsmittel erforderlich.

Die erfindungsgemäßen Formulierungen enthalten weiterhin mindestens einen direktziehenden Farbstoff.

Hierbei wurde festgestellt, dass der Farbeffekt einer Formulierung, die neben dem Farbstoffvorprodukt vom Indol- oder Indolin-Typ auch noch einen direktziehenden Farbstoff enthält, intensiver ist, als anhand der Farbeffekte der Einzelkomponenten, Farbstoffvorprodukt vom Indol- oder Indolin-Typ und direktziehender Farbstoff, zu erwarten gewesen wäre. Die Verdunklungseffekte werden durch die Kombination mit direktziehenden Farbstoffen synergistisch gesteigert.

Wie eingangs erwähnt, handelt es sich bei direktziehenden Farbstoffen um Farbstoffmoleküle, die direkt auf das Haar aufziehen und keinen oxidativen Prozess zur Ausbildung der Farbe mehr benötigen.

Bevorzugte direktziehende Farbstoffe umfassen Nitrophenylendiamine, Nitroaminophenole, Azofarbstoffe, Nitrofarbstoffe, Anthrachinone und Indophenole und sind vorzugsweise ausgewählt aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, Basic Yellow 57, Basic Yellow 87, HC Orange 1, Disperse Orange 3, Acid Orange 7, Basic Orange 31, HC Red 1, HC Red 3, HC Red 10, HC Red 11, HC Red 13, HC Red BN, Acid Red 33, Acid Red 52, Pigment Red 57:1, Basic Red 51, Basic Red 76, HC Blue 2, HC Blue 12, HC Blue 16, Disperse Blue 3, Acid Blue 7, Basic Blue 99, Acid Green 50, HC Violet 1, Disperse Violet 1, Disperse Violet 4, Acid Violet 43, Disperse Black 9, Acid Black 1, Acid Black 52, Basic Brown 16 und Basic Brown 17 sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure und deren Salze, 2-Amino-6-chlor-4-nitrophenol, 4-Ethylamino-3-nitrobenzoesäure und 2-Chlor-6-ethylamino-1-hydroxy-4-nitrobenzol.

Der direktziehende Farbstoff ist in der erfindungsgemäßen Formulierung üblicherweise in einer Menge von 0,001-5 Gew.-%, vorzugsweise 0,01-3 Gew.-%, bevorzugt 0,01-2 Gew.-%, bevorzugt 0,05-1,2 Gew.-%, bevorzugt in einer Menge von etwa 0,01 Gew.-%, 0,1 Gew.-%, 0,2 Gew.-%, 0,3 Gew.-%, 0,4 Gew.-%, 0,5 Gew.-%, 0,6 Gew.-%, 0,8 Gew.-%, 1,0 Gew.-%, 1,1 Gew.-%, 1,2 Gew.-%, 1,3 Gew.-%, 1,4 Gew.-%, 1,5 Gew.-%, 1,6 Gew.-%, 1,7 Gew.-%, 1,8 Gew.-%, 1,9 Gew.-%, 2,0 Gew.-%, jeweils bezogen auf das Gesamtgewicht der Formulierung, enthalten.

In einer bevorzugten Ausführungsform ist der direktziehende Farbstoff ausgewählt aus der Gruppe bestehend aus 2-Amino-6-chlor-4-nitrophenol, 1,4-Bis-(β-hydroxyethyl)-amino-2-nitrobenzol, Basic Yellow 57, Basic Red 76, Basic Brown 16, Basic Brown 17, Basic Blue 99, HC Blue 2 (2,2'-[[4-[(2-Hydroxyethyl)amino]-3-nitrophenyl]imino]bisethanol), HC Blue 12, HC Blue 16 und Acid Violet 43.

In einer besonders bevorzugten Ausführungsform der Erfindung handelt es sich bei dem direktziehenden Farbstoff um HC Blue 2.

In einer bevorzugten Ausführungsform enthält die erfindungsgemäße Formulierung 5,6-Dihydroxyindol als Farbstoffvorprodukt und HC Blue 2 als direktziehenden Farbstoff. Hierbei ist 5,6-Dihydroxyindol in einer Menge von 0,2-0,5 Gew.-% und der direktziehende Farbstoff in einer Menge von 0,01-2 Gew.-%, jeweils bezogen auf das Gesamtgewicht der Formulierung, enthalten.

Erfindungsgemäß enthält die kosmetische Formulierung mindestens einen aliphatischen Kohlenwasserstoff mit mindestens 10 Kohlenstoffatomen, der ggf. mit mindestens einer funktionellen Gruppe, ausgewählt aus -OH und -COOR, substituiert ist. Der aliphatische Kohlenwasserstoff ist bevorzugt ein gesättigter oder ungesättigter Kohlenwasserstoff, insbesondere bevorzugt ein gesättigter Kohlenwasserstoff. Der Kohlenwasserstoff hat bevorzugt 10-23, stärker bevorzugt 10-21 Kohlenstoffatome. Die Kohlenwasserstoffe gemäß Komponente (iii) sind bevorzugt unverzweigt.

Der aliphatische Kohlenwasserstoff (iii) hat bevorzugt 10-21 unverzweigte Kohlenstoffatome, ist gesättigt und mit -OH substituiert.

Bevorzugte Vertreter der Komponente (iii) sind Paraffine, Fettalkohole, Fettsäuren, Glycerinfettsäureester oder deren Salze.

Bevorzugte Paraffine (unsubstituierter Kohlenwasserstoff) sind C₁₀₋₅₀-Paraffine, stärker bevorzugt C₁₆₋₂₃-Paraffine.

Bevorzugte Fettalkohole (substituiert mit -OH) sind Laurylalkohol, Myristylalkohol, Cetylkalkohol, Margarylalkohol, Stearylalkohol, Cetearylalkohol und Behenylalkohol, insbesondere Laurylalkohol, Cetylalkohol, Cetearylalkohol und Stearylalkohol, insbesondere Cetearylalkohol.

Bevorzugte Fettsäuren (R=H) sind Caprinsäure, Laurinsäure, Myristinsäure, Palmitinsäure, Margarinsäure, Stearinsäure, Arachinsäure, Behensäure, Linolsäure, Linolensäure, Ölsäure, insbesondere Laurinsäure, Palmitinsäure und Stearinsäure, stärker bevorzugt Palmitinsäure und Stearinsäure.

Im Fall von R=Glycerinderivat, insbesondere Glycerinrest, Glycerinmonofettsäureesterrest oder Glycerindifettsäureesterrest, ist der substituierte Kohlenwasserstoff ein Glycerinfettsäureester. In der vorliegenden Erfindung bezieht sich der Begriff "Glycerinfettsäureester" auf einen Glycerinmono-, Glycerindi- oder Glycerintrifettsäureester. Glycerindifettsäureester weisen die Formel R'-COO-(CH₂CH(OH)CH₂)-OOCR' oder R'-COO-(CH₂CH(OOCR')CH₂)-OH auf. Glycerinmonofettsäureester weisen die Formel R'-COO-(CH₂CH(OH)CH₂)-OH oder HO-(CH₂CH(OOCR')CH₂)-OH auf. Dabei ist R' unabhängig voneinander der aliphatische Kohlenwasserstoff wie oben definiert. Glycerinmonofettsäureester enthalten eine Glycerin-Gruppe, welche über eine Esterbindung an eine einzige Fettsäure gebunden ist. In Glycerintrifettsäureestern sind alle Hydroxygruppen des Glycerins mit Fettsäuren verestert. Geeignete Beispiele sind Glycerinmonostearat, Glycerinmonobehenat, Glycerinmonocaprylat, Glycerinmonocaprat und Glycerinmonolaurat.

Bevorzugt ist Komponente (iii) mindestens ein Paraffin, Fettalkohol, eine Fettsäure, ein Glycerinfettsäureester oder eine Mischung davon, insbesondere mindestens ein gesättigter C₁₀₋₂₃-Fettalkohol, mindestens eine gesättigte C₁₀₋₂₃-Fettsäure oder eine Mischung davon, insbesondere mindestens ein gesättigter C₁₀₋₂₃-Fettalkohol.

Erfindungsgemäß macht der Anteil der Komponente (iii) mindestens 6 Gew.-%, bevorzugt 6-20 Gew.-%, bevorzugt 8-20 Gew.-%, bevorzugt 9-20 Gew.-%, stärker bevorzugt 8-17 Gew.-% bezogen auf die Gesamtmasse der kosmetischen Formulierung aus.

Bevorzugt ist das Massenverhältnis zwischen Komponenten ((i) + (ii)) zu Komponente (iii) im Bereich von 1:5 bis 1:70, stärker bevorzugt 1:10 bis 1:30.

Es hat sich überraschend gezeigt, dass bei Anwesenheit der Komponente (iii) in der kosmetischen Formulierung die Farbstoffvorprodukte und/oder die direktziehenden Farbstoffe schneller in die Keratinfaser einziehen. Dadurch wird die Farbtiefe gesteigert und gleichzeitig die Dauer der Färbeleistung gegenüber herkömmlichen Formulierungen verbessert. Zudem kann die Einwirkzeit verkürzt werden.

Dieser Effekt kann noch verstärkt werden, indem die erfindungsgemäße kosmetische Formulierung unter basischen Bedingungen auf die Keratinfaser aufgebracht wird. Bevorzugt hat die Formulierung daher einen pH-Wert im Bereich von pH 7-12, bevorzugt > 7-12, stärker bevorzugt 8-12 und am stärksten bevorzugt 9-11.

Darüber hinaus sorgt die Komponente (iii) für eine gute Haptik und die gewünschte Viskosität, um eine möglichst starke Haftung der kosmetischen Formulierung an der Keratinfaser zu bewirken.

Weiterhin kann die erfindungsgemäße kosmetische Formulierung mindestens ein eisenhaltiges Pigment (Komponente (iv)) enthalten. Bevorzugt ist das eisenhaltige Pigment CI 77499 (Eisenoxid schwarz). Komponente (iv) ist bevorzugt in einer Menge von 0,01 bis 20 Gew.-%, stärker bevorzugt 0,1 bis 7 Gew.-%, bezogen auf die Gesamtmasse der Formulierung enthalten.

Zur Verbesserung der Verarbeitbarkeit und Einstellung der Viskosität kann die kosmetische Formulierung ferner Wasser, insbesondere gereinigtes oder destilliertes Wasser, enthalten (Komponente (v)). Die kosmetische Formulierung enthält bevorzugt 50 bis 85 Gew.-%, stärker bevorzugt 70 bis 85 Gew.-%, Wasser bezogen auf die Gesamtmasse der Formulierung.

Weiterhin kann die erfindungsgemäße kosmetische Formulierung alle in solchen Zubereitungen bekannten Additive (d. h. Wirk-, Zusatz- und Hilfsstoffe) enthalten.

In einer Ausführungsform umfasst die kosmetische Formulierung ein Tensid. Das Tensid kann ein anionisches, nichtionisches, kationisches oder zwitterionisches Tensid sein. Bevorzugt ist das Tensid ein anionisches oder nichtionisches Tensid, insbesondere ein möglichst mildes (d.h. besonders hautverträgliches) anionisches oder nichtionisches Tensid. Nichtionische Tenside werden dabei erfindungsgemäß insbesondere wegen ihrer sehr guten Emulgationseigenschaften sowie ihrer ausgezeichneten Hautpflegeeigenschaften eingesetzt. Anionische Tenside sind bevorzugt, weil sie eine besonders hohe Reinigungsleistung aufweisen. Daher eignen sie sich besonders gut in Reinigungsformulierungen wie Shampoos. Kationische Tenside besitzen hervorragende Haarpflegeeigenschaften und werden erfindungsgemäß insbesondere in Haarpflegeformulierungen eingesetzt wie in Conditionern, Shampoos und Kuren.

Die erfindungsgemäße Formulierung enthält Tenside vorzugsweise in einer Menge von 2 bis 40 Gew.-%, insbesondere von 5 bis 30 Gew.-%, bevorzugt von 7 bis 20 Gew.-%, besonders bevorzugt von 10 bis 17 Gew.-%, jeweils bezogen auf das Gesamtgewicht der Formulierung. Geeignete Mengen an Tensid lauten: 8 Gew.-%; 9 Gew.-%; 10 Gew.-%; 11 Gew.-%; 12 Gew.-%; 13 Gew.-%; 14 Gew.-%; 15 Gew.-%; 16 Gew.-%; 17 Gew.-%; 18 Gew.-%; 19 Gew.-%; 20 Gew.-%; 21 Gew.-%; 22 Gew.-%; 23 Gew.-%; 24 Gew.-%; 25 Gew.-%, jeweils bezogen auf das Gesamtgewicht der Formulierung. Besonders bevorzugt enthält die kosmetische Formulierung der Erfindung ein oder mehrere anionische Tenside in einer Menge von 0,1 bis 20 Gew.-%, bevorzugt von 1 bis 17 Gew.-% und besonders bevorzugt von 5 bis 15 Gew.-%, jeweils bezogen auf das Gesamtgewicht der Formulierung. Geeignete Mengen an anionischem Tensid lauten: 1 Gew.-%; 2 Gew.-%; 3 Gew.-%; 4 Gew.-%; 5 Gew.-%; 6 Gew.-%; 7 Gew.-%; 8 Gew.-%; 9 Gew.-%; 10 Gew.-%; 11 Gew.-%; 12 Gew.-%; 13 Gew.-%; 14 Gew.-%; 15 Gew.-%; 16 Gew.-%; 17 Gew.-%; 18 Gew.-%, 19 Gew.-%, 20 Gew.-%, jeweils bezogen auf das Gesamtgewicht der Formulierung. In diesen Mengen weisen die Tenside eine besonders hohe Reinigungsleistung auf und sind äußerst verträglich für Haut, Kopfhaut und Haare.

In einer bevorzugten Ausführungsform ist das Tensid ein anionisches Tensid ausgewählt aus Alkylsulfonaten, Alkylsulfaten, Alkylethersulfaten, Alkylphosphaten, Alkylsarkosinaten, Alkyltauraten, Aminosäure-Tensiden und Mischungen davon. Besonders bevorzugt ist das Tensid ausgewählt aus Alkylsulfaten, Alkylsarkosinaten, Alkyltauraten, Alkylglutamat, wie Natriumcocoylglutamat/Dinatriumcocoylglutamat, Alkylgycinat, Alkylalaninat, wie Natriumcocoylalaninat und Mischungen davon. Ebenso bevorzugt wegen ihrer Reinigungsleistung sind Fettalkoholpolyglycerolethersulfate, Monoglyceridsulfate, Mono- und/oder Dialkylsulfosuccinate, Fettsäureisethionate, und α-Olefinsulfonate.

Alkylsulfate haben die generische Formel ROSO₃M, Alkylsarkosinate haben die generische Formel RC(O)N(CH₃)CH₂CO₂M, und Alkyltaurate haben die generische Formel RC(O)N(CH₃)CH₂CH₂SO₃M, wobei R jeweils ein C₄-C₂₆-Alkyl oder C₄-C₂₆-Alkenyl ist und M ein wasserlösliches Kation wie z.B. Ammonium, Natrium oder Kalium darstellt. Vorzugsweise ist M ein Natriumkation. Bevorzugt ist R ein C₁₂-C₁₆-Alkyl oder ein C₁₂-C₁₈-Alkyl.

In einer Ausführungsform ist das Tensid ein nichtionisches Tensid (auch als nichtionischer Emulgator bezeichnet). Nicht-einschränkende Beispiele umfassen Polyoxyethylenether eines oder mehrerer Fettalkohole, alkoxylierte Fettsäurealkylester, Polyglycerinether von Fettalkoholen, Polyglycerinester von Fettsäuren, Polyethylenglykol- und/oder Polypropylenglykolether, Fettsäureamide, Alkylphenolpolyglycolether, Aminoxide und Alkylpolyglucoside.

In einer Ausführungsform ist das Tensid ausgewählt aus der Gruppe der Polyoxyethylenether eines oder mehrerer Fettalkohole, Polyglycerinether von Fettalkoholen, Polyglycerinester von Fettsäuren und deren Mischungen.

Polyoxyethylenether sind Verbindungen der Formel R⁵(OC₂H₃)ₙOH, wobei R⁵ ausgewählt ist aus C₆-C₂₈-Alkyl, C₆-C₂₈-Alkenyl, substituierte und unsubstituierte Phenoxy-Gruppen; und n eine ganze Zahl größer 1 ist. Vorzugsweise wird der Polyoxyethylenether eines oder mehrerer Fettalkohole aus der Gruppe Steareth-2, Steareth-21, Makrogol-Cetostearylether 12, Ceteareth-25, Makrogol-Cetostearylether 20 und Mischungen der vorgenannten Verbindungen ausgewählt. Noch bevorzugter ist der Polyoxyethylenether eine Verbindung ausgewählt aus der Gruppe von Ceteareth-25, Makrogol-Cetostearylether 20 und Mischungen der vorgenannten Verbindungen.

Der Begriff "Polyglycerinether von Fettalkoholen" bezieht sich auf eine Verbindung der Formel R⁶O-(C₃H₆O₂)ₙ-H, wobei R⁶ ein verzweigtes oder lineares C₆-C₂₈-Alkyl oder C₆-C₂₈-Alkenyl ist und n eine ganze Zahl größer als 1, vorzugsweise eine ganze Zahl von 2 bis 10, ist. Es wird bevorzugt, dass die Formulierung 0,01 bis 15,0 Gew.-%, 0,1 bis 10,0 Gew.-% oder 1 bis 5,0 Gew.-% Polyglycerinether enthält.

Der Begriff "Polyglycerinester von Fettsäuren" bezieht sich auf Verbindungen, die sowohl eine Polyglycerineinheit als auch mindestens eine C₆-C₂₆-Alkyl- oder C₆-C₂₆-Alkenylcarbonsäureeinheit enthalten. Diese Verbindungen können die Formel R⁷-R⁸-(C₃H₆O₂)ₙ-H aufweisen, wobei R⁷ ein C₆-C₂₆-Alkanoat- oder C₆-C₂₆-Alkenoat-Rest ist und R⁸ ein geeignetes Verbindungsmolekül oder eine direkte Bindung ist. Somit können die Polyglycerineinheit und die C₆-C₂₆-Alkyl- oder C₆-C₂₆-Alkenylcarbonsäureeinheit direkt durch eine Esterbindung verbunden sein oder eine Verbindungseinheit enthalten, die diese beiden Einheiten miteinander verbindet. Nicht einschränkende Beispiele für diese Gruppe sind Polyglyceryl-3-methylglucosedistearat, Polyglycerinpolycrinoleat, Polyglyceryl-Dimerat-Isostearat, Polyglyceryl-2-Laurat, Polyglyceryl-2-Sesquiisostearat, Polyglyceryl-3-Distearat (Cremophor GS 32), Polyglyceryl-3-Oleat, Polyglyceryl-3-Methylglykose-Distearat, Polyglyceryl-4-Caprat (Polyglycerolcaprat T2010190), Polyglyceryl-4-Diisostearat / Polyhydroxystearat / Sebacat (Isolan GPS) und Polyglyceryl-4-Isostearat.

In einer Ausführungsform umfasst das Tensid ein kationisches Tensid, wie beispielsweise ein quaternäres Tensid. Quaternäre Tenside enthalten mindestens ein N-Atom, das mit 4 Alkyl- oder Arylgruppen kovalent verbunden ist. Dies führt, unabhängig vom pH-Wert, zu einer positiven Ladung. Vorteilhaft sind Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

In der vorliegenden Erfindung ist das Tensid wie oben definiert unterschiedlich zu dem aliphatischen Kohlenwasserstoff der Komponente (iii).

In einer weiteren Ausführungsform enthält die erfindungsgemäße Formulierung mindestens ein Additiv. Bevorzugt sind Additive, die üblicherweise in Shampoos, Conditionern und Emulsionen zur Behandlung der Haut, der Kopfhaut und der Haare eingesetzt werden. Das mindestens eine Additiv kann in einem Anteil von 0,01 Gew.-% bis 12,0 Gew.-%, mehr bevorzugt von 0,25 Gew.-% bis 10,0 Gew.-%, insbesondere von 1,0 bis 7,0 Gew.-% vorliegen.

Das mindestens eine Additiv kann ferner ausgewählt sein aus der Gruppe bestehend aus Haarkonditionierungsmitteln, Rückfettern, Konservierungsmitteln, Stabilisatoren, Duftstoffen, Antioxidantien, Rheologiemodifikatoren, Verdickungsmitteln, Pflegemitteln, Farbstoffen, Perlganzmitteln, Aufhellungsmitteln, Lösungsmitteln, festigenden Verbindungen, Antischuppenwirkstoffen, Vitaminen, Alkalisierungsmitteln bzw. pH-Stellmitteln und Kombinationen hiervon.

Haarkonditionierungsmittel können statische Aufladungen der Haare durch das Neutralisieren von elektrischer Ladung an ihrer Oberfläche verringern. Beispiele für Haarkonditionierungsmittel sind quartäre Ammoniumverbindungen.

Rückfetter, auch Rückfettungsmittel oder Überfettungsmittel genannt, sind lipophile Substanzen, die eine störende Auswirkung auf die epidermale Barrierefunktion verhindern können. Beispiele für Rückfetter sind Wollwachs, Squalen, flüssiges Paraffin, pflanzliche Öle, Silikone und Cetylpalmitat.

Konservierungsmittel sind Substanzen, die zur Konservierung verwendet werden, indem sie die Formulierung zersetzende Mikroorganismen abtöten und/oder deren Wachstum hemmen. Vorzugsweise können die Konservierungsmittel ausgewählt sein aus der Gruppe bestehend aus Benzoesäure, Benzoesäurederivaten, Sorbinsäure, Sorbinsäurederivaten, Salicylsäure, Salicylsäurederivaten, Phenoxyethanol, Parabenen und Kombinationen hiervon. In einer bevorzugten Ausführungsform werden Natriumbenzoat und/oder Kaliumsorbat als Konservierungsmittel in der erfindungsgemäßen Formulierung eingesetzt. Natriumbenzoat setzt in leicht saurem Milieu Benzoesäure und Kaliumsorbat Sorbinsäure frei. Beiden Säuren wird eine antimikrobielle Wirkung zugeschrieben.

Stabilisatoren können lichtempfindliche Komponenten gegenüber Strahlung schützen und sind bevorzugt UV-Absorber wie beispielsweise Benzophenonderivate.

Die Zugabe von Duftstoffen kann für einen angenehmen Geruch der Formulierung sorgen. Beispiele sind die dem Fachmann bekannten Parfums.

Ein Antioxidans oder Antioxidationsmittel ist eine chemische Verbindung, die eine Oxidation anderer Komponenten in der erfindungsgemäßen Formulierung verlangsamt oder gänzlich verhindert. Als Antioxidantien kommen beispielsweise Zitronensäure, Ascorbinsäure, Natriumsulfit und Butylhydroxyanisol in Frage.

Rheologiemodifikatoren und Verdickungsmittel können dabei helfen, die Applikationseigenschaften der erfindungsgemäßen Formulierung zu verbessern. Als Rheologiemodifikator und Verdickungsmittel kann die Zugabe von Kochsalz (Natriumchlorid) in Betracht gezogen werden. Durch die Zugabe von Kochsalz kann die Fließfähigkeit der erfindungsgemäßen Formulierung in gewissen Grenzen beeinflusst und auf das nötige Maß eingestellt werden. Als Verdicker können zusätzlich natürlich vorkommende Gelbildner eingesetzt werden, die bevorzugt ausgewählt sind aus Agar, Xanthan Gum, Cellulose und oder CelluloseDerivaten oder Alginsäure.

Im Rahmen der Anmeldung sollen unter Pflegemitteln Substanzen verstanden werden, welche die Haare und/oder die Kopfhaut pflegen. Hydrolysiertes Weizenprotein, Panthenol und Allantoin wirken pflegend auf Kopfhaut und Haare. Hydrolysiertes Weizenprotein hat hauptsächlich feuchtigkeitsspendende Eigenschaften.

Als weitere Farbstoffe können in der erfindungsgemäßen Formulierung Anfärbestoffe enthalten sein, beispielsweise um der Formulierung eine optisch ansprechende Farbe zu geben. In der erfindungsgemäßen Formulierung kann z.B. CI 47005 als geeigneter Anfärbestoff enthalten sein.

Als Lösungsmittel kann ein für den Fachmann auf diesem Gebiet gängiges Lösungsmittel oder Lösungsmittelgemisch eingesetzt werden. Bevorzugte Lösungsmittel sind Ethanol oder Butylenglykol, insbesondere 1,4-Butylenglykol, Propylenglykol und Isopropylalkohol. Bevorzugt ist Ethanol bzw. Propylenglykol. Diese Lösungsmittel können vorzugsweise in einer Menge von 0,1 bis 70 Gew.% in der erfindungsgemäßen Formulierung enthalten sein. Ganz besonders bevorzugt sind sie in einer Menge von 0,1 bis 5,0 Gew.% enthalten.

Festigende Verbindungen sind vorzugsweise aus der Gruppe bestehend aus Wachsen, synthetischen Polymeren und Mischungen davon ausgewählt. Geeignete festigende Verbindungen sind dem Fachmann bekannt und beispielsweise in Goddard, E. Desmond; Gruber, James V. (1999), "Principles of polymer science and technology in cosmetics and personal care", Series: Cosmetic science and technology series : v. 22., New York (Marcel Dekker, INC.); Schrader, Karlheinz (1989), "Grundlagen und Rezepturen der Kosmetika" (2. Auflage), Kapitel 3.7, Heidelberg (Hüthig); sowie Scott, Richard (2017), "ingredients focus:cosmetic waxes and butters", Personal Care Europe 10(3), 46-47 beschrieben.

Antischuppenwirkstoffe werden eingesetzt, um übermäßige Bildung von Schuppen auf der Kopfhaut zu kontrollieren. Geeignete Antischuppenwirkstoffe sind dem Fachmann bekannt und beispielsweise in Trüeb, R. M. (2009). "Kopfschuppen erfolgreich behandeln." Akt Dermatol 35(01/02): 19-24; Trueb, R. M. (2007). "Shampoos: ingredients, efficacy and adverse effects." J. Dtsch. Dermatol. Ges. 5(5): 356-365; Sanfilippo, A. and J. English (2006). "An overview of medicated shampoos used in dandruff treatment." P AND T 31(7): 396; sowie Futterer, E. (1981). "Evaluation of efficacy of antidandruff agents." J Soc Cosmet Chem 32: 327-338 beschrieben.

Vitamine und Mineralstoffe wie Niacinamid (Vitamin B3), Vitamin E oder Zink werden eingesetzt, um einen positiven Effekt auf das Wachstum und die Reparatur von Haarschädigungen zu erzielen. Geeignete Vitamine und Mineralstoffe sind dem Fachmann bekannt und beispielsweise in Pietrzik K, Golly I, Loew D, Handbuch Vitamine, Elsevier GmbH, Urban & Fischer Verlag, München 2008; H. Lautenschläger, Vitamine in der Kosmetik, medical Beauty Forum 2020 (3), 14-17; sowie Martini M.C., Chivot M., Peyrefitte G., Lehrbuch Kosmetik: Grundlagen - Grundstoffe - Grundtechniken, Hogrefe AG, Bern 2001 beschrieben.

Alkalisierungsmittel bzw. pH-Stellmittel werden eingesetzt, um den pH-Wert der Formulierung auf einen gewünschten Bereich einzustellen. Geeignete Alkalisierungsmittel bzw. pH-Stellmittel umfassen unter anderem Natriumhydroxid, Monoethanolamin 0,1-5%, Phosphorsäure und Citronensäure.

Erfindungsgemäße Zusammensetzungen sind bevorzugt Booster-Pflegeprodukte.

Es hat sich überraschenderweise gezeigt, dass das Eigenschaftsprofil der erfindungsgemäßen Formulierung besonders gut geeignet ist, kurze und/oder wenig beanspruchte Keratinfasern, wie z.B. Haare an der Schläfe oder Barthaare, zu behandeln, deren Färbung üblicherweise als problematisch gilt.

Die kosmetische Formulierung der vorliegenden Erfindung ist insbesondere zur Pigmentierung und/oder Repigmentierung von Keratinfasern geeignet.

In einem Aspekt betrifft die Erfindung daher auch die Verwendung der erfindungsgemäßen Formulierung zum Färben von Keratinfasern, insbesondere von Haaren, bevorzugt Barthaaren oder Haaren an den Schläfen.

Unter "Keratinfasern" sind im Sinne der vorliegenden Erfindung Haare zu verstehen, insbesondere menschliche Haare. Der Begriff "menschliche Haare" umfasst hierbei insbesondere Kopfhaare und Barthaare.

Die kosmetische Formulierung der vorliegenden Erfindung kann weiterhin in Kombination mit mindestens einer zusätzlichen kosmetischen Formulierung verwendet werden. Üblicherweise ist die zusätzliche kosmetische Formulierung, die von der erfindungsgemäßen Formulierung unterschiedlich ist, ein Shampoo, ein Conditioner, eine Pflegekur oder ein Tonikum. Bevorzugt enthält die zusätzliche kosmetische Formulierung Aktivstoffe, insbesondere Xanthinderivate wie z.B. Koffein und/oder Farbstoffvorprodukt (wie oben definiert).

Conditioner, Shampoos, Pflegekuren, etc. sind dem Fachmann bekannt. Besonders bevorzugt wird die erfindungsgemäße kosmetische Formulierung in Kombination mit der in EP 4 154 864 beschriebenen kosmetischen Formulierung verwendet.

Entsprechend betrifft die vorliegende Erfindung auch ein Kit, umfassend
(a) eine kosmetische Formulierung der vorliegenden Erfindung und
(b) eine kosmetische Formulierung, die insbesondere ein Xanthinderivat und/oder Farbstoffvorprodukt (wie oben definiert) enthält.

Komponenten (a) und (b) sind unterschiedliche kosmetische Formulierungen.

Erfindungsgemäß wird die Zusammensetzung topisch angewendet. Unter einer topischen Anwendung ist eine äußerliche Anwendung, insbesondere eine örtliche, äußerliche Anwendung, insbesondere direkt auf die Keratinfaser, zu verstehen.

In einem weiteren Aspekt betrifft die vorliegende Erfindung ein Verfahren zum Färben von Keratinfasern, umfassend die Schritte
(1) Aufbringen der kosmetischen Formulierung der vorliegenden Erfindung auf die Keratinfasern, insbesondere auf Barthaar oder Schläfen,
(2) Einwirken der kosmetischen Formulierung an der Luft und ggf. Umsetzen des Farbstoffvorprodukts zum Farbstoff mit Oxidationsmitteln und
(3) Spülen der nach Schritt (2) behandelten Keratinfasern mit Wasser.

Bevorzugt wird die kosmetische Formulierung der vorliegenden Erfindung im Schritt (1) auf das wasserfeuchte oder trockene Haar verteilt. Dies kann beispielsweise mit einem Pinsel oder einer Bürste (die ggf. an dem die Formulierung enthaltenden Behälter angebracht ist) erfolgen.

Im Schritt (2) lässt man die erfindungsgemäße Formulierung bevorzugt 1-30 Minuten, stärker bevorzugt 2-20 Minuten, stärker bevorzugt 1-5 Minuten einwirken.

Nach Schritt (2) wird die so behandelte Keratinfaser mit Wasser gespült, sodass bevorzugt alle Bestandteile der erfindungsgemäßen Formulierung nach Schritt (3) entfernt sind.

In einer bevorzugten Ausführungsform können die Keratinfasern vor Schritt (1) oder nach Schritt (3) mit einer zusätzlichen kosmetischen Formulierung, bevorzugt einem Shampoo, einem Conditioner oder einer Pflegekur, behandelt werden. Die zusätzliche kosmetische Formulierung ist wie oben beschrieben.

Anhand der nachfolgenden Formulierungen soll die Erfindung verdeutlicht werden, ohne sie jedoch auf die speziellen Beispiele einschränken zu wollen.

### Beispielrezeptur 1

| Inhaltsstoff | Gew.-% |
|---|---|
| Wasser | ad 100 |
| Niacinamid | 0,05 |
| Cetearyl Alcohol | 12 |
| Stearinsäure | 2,3 |
| Palmitinsäure | 2,2 |
| Natriumlaurylsulfat | 1 |
| HC Blue 2 | 0,2 |
| Monoethanolamin | 3 |
| 5,6-Dihydroxyindole | 0,4 |
| Zink PCA | 0,05 |
| pH | 10±0,8 |

### Beispielrezeptur 2

| Inhaltsstoff | Gew.-% |
|---|---|
| Wasser | ad 100 |
| Niacinamid | 0,05 |
| Cetearyl Alcohol | 8 |
| Stearinsäure | 2,3 |
| Palmitinsäure | 2,2 |
| Natriumlaurylsulfat | 1 |
| HC Blue 2 | 0,2 |
| Monoethanolamin | 3 |
| 5,6-Dihydroxyindole | 0,5 |
| Zink PCA | 0,05 |
| pH | 10±0,8 |

### Beispielrezeptur 3

| Inhaltsstoff | Gew.-% |
|---|---|
| Wasser | ad 100 |
| Niacinamid | 0,05 |
| Cetearyl Alcohol | 15 |
| Stearinsäure | 2,3 |
| Palmitinsäure | 2,2 |
| Natriumlaurylsulfat | 1 |
| Monoethanolamin | 3 |
| HC Blue 2 | 0,2 |
| 5,6-Dihydroxyindole | 0,3 |
| Zink PCA | 0,05 |
| pH | 10±0,8 |

## Patentansprüche

1. Kosmetische Formulierung enthaltend
(i) mindestens ein Farbstoffvorprodukt ausgewählt aus der Gruppe bestehend aus Indolin-Derivaten und Indol-Derivaten,
(ii) mindestens einen direktziehenden Farbstoff und
(iii) mindestens einen aliphatischen Kohlenwasserstoff mit mindestens 10, bevorzugt 10-50, Kohlenstoffatomen, der ggf. mit mindestens einer funktionellen Gruppe ausgewählt aus -OH und -COOR substituiert ist, wobei R unabhängig voneinander H, ein Glycerinderivat oder ein physiologisch verträgliches Kation ist,
wobei die Komponente (iii) bevorzugt mindestens 6 Gew.-%, stärker bevorzugt 8-20 Gew.-%, bezogen auf die Gesamtmasse der kosmetischen Formulierung ausmacht.

2. Kosmetische Formulierung nach Anspruch 1, wobei das Farbstoffvorprodukt ein Indolderivat, insbesondere ein 5,6-Dihydroxyindol-Derivat gemäß Formel (I) 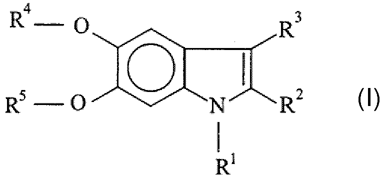
oder ein physiologisch verträgliches Salz davon ist, wobei, jeweils unabhängig voneinander,
R¹ Wasserstoff, eine C₁-C₄-Alkylgruppe oder eine C₁-C₄-Hydroxy-alkylgruppe bedeutet,
R² Wasserstoff oder eine -COOH-Gruppe bedeutet, wobei die -COOH-Gruppe auch als Salz mit einem physiologisch verträglichen Kation vorliegen kann,
R³ Wasserstoff oder eine C₁-C₄-Alkylgruppe bedeutet,
R⁴ Wasserstoff, eine C₁-C₄-Alkylgruppe, eine Aminogruppe oder eine Gruppe - COR⁶ bedeutet, in der R⁶ eine C₁-C₄-Alkylgruppe bedeutet, und
R⁵ eine der unter R⁴ genannten Gruppen bedeutet.

3. Kosmetische Formulierung nach Anspruch 1, wobei das Farbstoffvorprodukt ein Indolinderivat, insbesondere ein 5,6-Dihydroxyindolin-Derivat gemäß Formel (II) 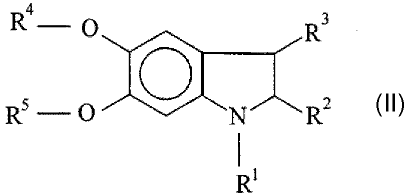
oder ein physiologisch verträgliches Salz davon ist, wobei, jeweils unabhängig voneinander,
R¹ Wasserstoff, eine C₁-C₄-Alkylgruppe oder eine C₁-C₄-Hydroxy-alkylgruppe bedeutet,
R² Wasserstoff oder eine -COOH-Gruppe bedeutet, wobei die -COOH-Gruppe auch als Salz mit einem physiologisch verträglichen Kation vorliegen kann, R³ Wasserstoff oder eine C₁-C₄-Alkylgruppe bedeutet,
R⁴ Wasserstoff, eine C₁-C₄-Alkylgruppe, eine Aminogruppe oder eine Gruppe - COR⁶ bedeutet, in der R⁶ eine C₁-C₄-Alkylgruppe bedeutet, und
R⁵ eine der unter R⁴ genannten Gruppen bedeutet.

4. Kosmetische Formulierung nach einem der vorangehenden Ansprüche, wobei die Komponente (i) mindestens 0,2 Gew.-%, bevorzugt 0,2-0,5 Gew.-%, bezogen auf die Gesamtmasse der kosmetischen Formulierung ausmacht.

5. Kosmetische Formulierung nach einem der vorangehenden Ansprüche, wobei der direktziehende Farbstoff ein Nitrophenylendiamin, Nitroaminophenol, Nitrofarbstoff, Azofarbstoff, Anthrachinon oder Indophenol ist, und wobei die Komponente (ii) bevorzugt 0,01-2 Gew.-% bezogen auf die Gesamtmasse der kosmetischen Formulierung ausmacht.

6. Kosmetische Formulierung nach einem der vorangehenden Ansprüche, wobei der aliphatische Kohlenwasserstoff (iii) 10-21 unverzweigte Kohlenstoffatome aufweist, gesättigt ist und mit -OH substituiert ist.

7. Kosmetische Formulierung nach einem der vorangehenden Ansprüche, wobei die Formulierung einen pH-Wert im Bereich von pH 7-12, bevorzugt >7-12, stärker bevorzugt 8-12, am stärksten bevorzugt 9-11, aufweist.

8. Kosmetische Formulierung nach einem der vorangehenden Ansprüche, ferner umfassend
(iv) mindestens ein eisenhaltiges Pigment, bevorzugt in einer Menge von 0,01-20 Gew.-% bezogen auf die Gesamtmasse der Formulierung, und
(v) ggf. Wasser, bevorzugt in einer Menge von 50-85 Gew.-% bezogen auf die Gesamtmasse der Formulierung.

9. Verwendung der kosmetischen Formulierung nach einem der Ansprüche 1-8 zum Färben von Keratinfasern, insbesondere von Haaren.

10. Verwendung nach Anspruch 9, wobei die Haare Barthaare oder Haare an den Schläfen sind.

11. Verwendung nach einem der Ansprüche 9 und 10, in Kombination mit mindestens einer zusätzlichen kosmetischen Formulierung, die insbesondere ein Xanthin-Derivat und/oder ein Farbstoffvorprodukt enthält.

12. Kit umfassend
(a) eine kosmetische Formulierung nach einem der Ansprüche 1-8 und
(b) eine kosmetische Formulierung, die insbesondere ein Xanthin-Derivat und/oder ein Farbstoffvorprodukt enthält.

13. Verfahren zum Färben von Keratinfasern, umfassend die Schritte
(1) Aufbringen der kosmetischen Formulierung nach einem der Ansprüche 1-8 auf die Keratinfasern, insbesondere auf Barthaar oder Schläfen,
(2) Einwirken der kosmetischen Formulierung an der Luft und ggf. Umsetzen des Farbstoffvorprodukts zum Farbstoff mit Oxidationsmitteln, und
(3) Spülen der nach Schritt (2) behandelten Keratinfasern mit Wasser.

14. Verfahren nach Anspruch 13, wobei Schritt (1) durch Aufbringen mit einem Pinsel erfolgt.

15. Verfahren nach Anspruch 13 oder 14, wobei Schritt (2) 1-5 Minuten dauert.

16. Verfahren nach einem der Ansprüche 13-15, wobei die Keratinfasern vor Schritt (1) oder nach Schritt (3) mit einer zusätzlichen kosmetischen Formulierung, die insbesondere ein Xanthin-Derivat und/oder ein Farbstoffvorprodukt enthält, behandelt werden.
